Europäisches Patentamt

European Patent Office

Office européen des brevets

(11) Publication number: **0 378 921**
**A2**

## EUROPEAN PATENT APPLICATION

(21) Application number: **89313255.5**

(22) Date of filing: **19.12.89**

(51) Int. Cl.⁵: **C12N 5/04, //C12Q1/28**

The title of the invention has been amended (Guidelines for Examination in the EPO, A-III, 7.3).

(30) Priority: **21.12.88 US 287859**

(43) Date of publication of application:
**25.07.90 Bulletin 90/30**

(84) Designated Contracting States:
**AT BE CH DE ES FR GB GR IT LI LU NL SE**

(71) Applicant: **IDAHO RESEARCH FOUNDATION, INC.**
**PO Box 9645**
**Moscow Idaho 83843(US)**

(72) Inventor: **Crawford, Donald L.**
**1988 Appaloosa Road**
**Moscow Idaho 83843(US)**
Inventor: **Shetty, Kalidas, 301 Luminous**
**Kannon Dai,Ichibankan, 1-34-16 Kannon Dai**
**Tsukuba, Ibasaki305(JP)**
Inventor: **Korus, Roger A.**
**1038 Virginia Avenue**
**Moscow Idaho 83843(US)**

(74) Representative: **Harrison, David Christopher et al**
**MEWBURN ELLIS 2 Cursitor Street**
**London EC4A 1BQ(GB)**

(54) **Enhancement of plant metabolite production by timed elicitation.**

(57) Methods are provided for maximizing the production of secondary metabolites produced from plant cell cultures. The methods involve monitoring the physiological state of the culture cells so that elicitors, which stimulate metabolite production, can be applied at an optimum time. This enhanced production of metabolites following elicitation can be further maximized by the addition of cell viability stabilizers and/or nutrients at the time of elicitation.

EP 0 378 921 A2

# MAXIMIZED ENHANCEMENT OF PLANT METABOLITE PRODUCTION BY TIMED ELICITATION

This invention relates to the production of metabolites from cell cultures.

Plants are increasingly important sources of chemicals used in medicine, fruit flavors and aromas, cosmetics and agrochemicals. Most of these chemicals are secondary metabolites such as phenolic compounds and alkaloids. Interest in these compounds has heightened as their beneficial effects are proven. For example, flavones have been shown to participate in binding to biopolymers (enzymes) and cell membranes, complexing of heavy metal ions, and electron transfer in enzyme systems, e.g. oxidoreductases, and free radical scavenging. Flavones also possess marked antiallergic activity. These metabolites have also demonstrated antihaemorrhagic, circulation-promoting, antiphlogistic, and cardiovascular activities.

Most of these chemicals are extracted directly from plant tissues. However, there is increasing interest in producing these metabolites in plant cell culture. The advantages of cell culture over the conventional agricultural production of plant material are numerous. The production of botanical products would be possible independent of environmental factors, such as climate, plant pests, and seasons, and it would be possible to produce them at any place on earth. Also, the quality of the raw products could be produced at a uniformity never observed with field grown material. Further, any newly discovered beneficial plants could be put immediately into culture circumventing the need for lengthy agricultural propagation and cultivation. However, the criterion which governs utilization of these techniques in industry is economic. Only if the product under consideration can be produced industrially by cell culture techniques at a cost equal to or lower than field-produced goods will the biotechnological realization of the cell culture method advance.

Plant cells cannot compete with true microorganisms, like bacterial or fungal cells, for the production of primary metabolites; growth and metabolic rates of plant cells are too slow compared to these organisms. The opportunity for an industrial application of plant cell culture will almost exclusively lie in the production of the specific and valuable low or high molecular weight products which are synthesized only by higher plants. For the most part, the potential for the chemical synthesis of these so-called secondary compounds is not realized.

In order to realize the industrial application of plant cell culture for the production of useful secondary metabolites, it is essential to enhance the rates of biosynthesis. Towards this goal, selection mechanisms have been utilized to identify cell lines with improved biosynthetic capabilities. These high yielding strains must be stable to be useful for the industrial production of metabolites by the cell culture system. Further, factors which influence the growth and product formation of cell cultures such as natural or synthetic plant hormones or effectors, have to be considered.

Attempts to increase metabolite production have been attempted by modifying culture conditions. One such method to increase secondary metabolites calls for culturing plant cells, first in a liquid medium suited to the growth of the cells, with a subsequent culturing step in modified liquid medium suited to the production of secondary metabolites. This method is both time consuming and laborious. The present invention is drawn to methods for the maximization of metabolite production from cell cultures.

Relevant Literature

U.S. Patent, 4,717,664, discloses a method for producing secondary metabolites of higher plants using suspension cultures of undifferentiated cells. The cultures are carried out in at least two stages in liquid media. The article "Rapid transient induction of phenylalanine ammonia-lyase mRNA in elicitor-treated bean cells" by Edwards, et al, Proc. Nat. Acad. of Sci., USA (1985) 82:6731-6735 discloses that elicitor-treated cells of Phaseolus vulgaris show a rapid stimulation of phenylalanine ammonia-lyase mRNA synthesis as an early event in the defense response leading to accumulation of phenylpropanoid-derived phytoalexins. The article "Phytoalexin synthesis in soybean cells: elicitor induction of phenylalanine ammonia-lyase and chalcone synthase mRNAs and correlation with phytoalexin accumulation" by Ebel, et al in Archives of biochemistry and biophysics (1984) 232: 240-248 discloses that fungal elicitors induce large and rapid increases in the activities of enzymes of general phenylpropanoid metabolism, phenylalanine ammonia-lyase, and of the flavonoid pathway, acetyl-CoA carboxylase and chalcone synthase in suspension cultured soybean cells.

## SUMMARY OF THE INVENTION

Methods for maximizing the levels of secondary metabolites produced from cell cultures are provided. The methods involve monitoring the physiological state of the culture cells so that elicitors, which stimulate metabolite production, can be applied at an optimum time. Enzyme assays for extracellular products can be used to indicate the optimum time for the addition of elicitors. This enhanced production of secondary metabolites following elicitation can be further maximized by the addition of cell viability stabilizers and/or nutrients at the time of elicitation.

## DESCRIPTION OF THE SPECIFIC EMBODIMENTS

In accordance with the present invention, methods are provided for maximizing the production of secondary metabolites from cell cultures. Elicitors, which stimulate metabolite production, are utilized to produce economically viable levels of specific products by maximizing their production at an optimal time in the growth phase of a plant cell suspension culture. By carefully timing the addition of elicitors, secondary metabolite production is enhanced and thus, increased metabolite yield by cells can be achieved in significantly shortened periods of time, as compared to normal growth conditions. For example, the instant Experimental section demonstrates that secondary metabolite production in a 48 hour period can be, at least, doubled by the addition of an elicitor to the culture medium at an optimum time, from about 67.4 mg/l to at least 137.3 mg/l (Table 2). Further, by adding cell viability stabilizers and/or nutrients at the time of elicitation the increased metabolite production can be stabilized rendering the metabolite production system more efficient and economic. The addition of elicitors induces large and rapid increases in the activities of enzymes of general phenylpropanoid metabolism and of the flavonoid pathway. This sudden increase in metabolism can have inhibitory or deleterious effects on the plant cells. The addition of cell viability stabilizers and/or nutrients at the time of elicitation function to maintain and extend cell viability following product formation after eilcitation.

Cell cultures of the present invention are plant cell cultures. Cells from any plant species which are capable of growth or proliferation in culture may be used. The particular plant culture will be chosen based upon the metabolite of interest. Thus, cell cultures which are suitable for use include any which produce the metabolite of interest and respond to elicitation. Species of plants which may be utilized are not limited, and may include, among others, plants from Juglandaceae, Boraginaceae, Pyrolaceae, Plumbaginaceae, Lytharaceae, Solanaceae, and Ranunculaceae.

The culture medium utilized is any which is suited to the growth and proliferation of cells including Linsmaier-Skoog medium, Murashigae-Skoog medium, Gamborg et al.'s B-S medium and the like. It is recognized that a wide choice of media is available and many modifications in the culture medium are possible. Changes in hormones, vitamins or other substituents or their concentrations are examples of possible variations.

For the most part, the plant cells will be callus, gall, or undifferentiated cells. Several methods exist for obtaining undifferentiated cells. In general, tissue samples from a plant part such as roots, leaves, stems, seeds, or other parts are sterilized and added to a solid medium where a part of the tissue proliferates into a mass of undifferentiated cells or callus. These undifferentiated cells provide an inoculant for suspension cultures. The present invention can be used with any of the wide variations which are possible in cell culture work; in the types of cells used, the tissue from which the cells are derived and the medium used to grow the cells.

Selected plant cells can be grown by any of the fermentation techniques known for plant cells. Various reactor systems include the airlift reactor, shake flask, turbine impellers, draft tube with Kaplan turbine and the like. Because of their size, plant cells are sensitive to shear stress and special care is needed with highly viscous fermentation. Additional care must also be taken for sterility of the system prior to the onset of elicitation

Plant cells can be grown in uniform well-defined sterile medium on a large scale. Large populations of plant cells can therefore be obtained rapidly in a relatively uniform state. Plant cells in culture do not necessarily exhibit any of the patterns of development observed for cells in intact whole plants. Rather, plant cells in culture are in a unique developmental state. For example, different isozymes of aspartate kinase are produced by carrot cells during different phases of culture growth (Davies et al. Plant Physiol. (1978) 61, Abstr. 526) and variations in the expression of peroxidase isozymes have been noted for cells in different clump sizes (Verna et al. Can. J. Biochem. (1970) 48:444-449). The present invention recognizes the differences in metabolic activities such that elicitation of secondary metabolites occurs at the optimum time in the culture cycle.

The physiological state of the culture is monitored to determine the optimum time for the addition of elicitors. By physiological state is intended the phase of growth of the cell culture, i.e. stationary, exponential, etc. The optimum time for the addition of elicitors to the culture is approximately the end of the exponential growth phase. That is, the optimum time is the time in which the cells have optimum enzyme potential as well as cell viability for responding to elicitors. Methods to determine the optimum time for the addition of elicitors includes measuring biomass production, measuring carbohydrate utilization, and assaying for extracellular products. The production of biomass or cell dry weight, is a good indicator of cell growth. A constant biomass for a period of about 1 day generally indicates that cells have reached approximately the end of the exponential growth phase and have entered the stationary growth phase. Thus, about the end of exponential growth phase is defined as the period when the culture biomass is constant for a time of about 12 hours to about 2 days usually about 1 day. In this manner, direct measurements of biomass can be performed to determine the growth phase of the culure.

A declining growth rate is also reflected in a declining rate of carbohydrate utilization. Thus, monitoring levels of carbohydrate utilization could also be used to determine the optimum time for the addition of elicitors.

However, it has been determined that assays for extracellular products which parallel cell growth, such as extracellular peroxidase activity, provide alternative means to determine the physiological growth phase of cultures. Assaying for extracellular peroxidase activity provides a particularly efficient, sensitive and economical means to measure biomass, i.e., culture growth. Extracellular peroxidase activity closely parallels growth in cell cultures as demonstrated in Arachis hypogea, Nicotiana tobaccum and Glycine max. Thus, the correlation between cell dry weight and extracellular peroxidase activity provides a novel alternative means to measure biomass production indirectly by measuring peroxidase activity.

The present method calls for maximizing levels of metabolites from cell cultures. By maximizing is intended, the production of a higher concentration of metabolites in a shortened period of time as compared to normal growth conditions. That is, the time during which the optimum quantity of secondary metabolites is produced is reduced by the addition of elicitors into the cell cultures at an optimum time. Phenolic yields in the range of about 0.12 g/l to about 0.15 g/l can be realized using elicitation. Even greater enhancement of metabolite production can be attained by a combination of timed elicitation, addition of cell viability stabilizers and/or the addition of nutrients at the time of elicitation.

Elicitation is an induction process which results in increases in the levels of mRNA for phenylalanine ammonia-lyase (PAL), chalcone synthase, chalcone isomerase, etc. being the first responses to elicitor contact in a cascade of biochemical events. This induction process includes increased activities of enzymes of general phenylpropanoid metabolism, of flavonoid biosynthesis, of pterocarpan biosynthesis, of defense response, etc. Included within the induction process triggered by elicitors is the synthesis of low molecular weight secondary metabolites, i.e., aromatic compounds of about 100 to about 1,000 daltons (d). The maximum yield of metabolites which result from elicitation is generally found after about 10 hours to about 5 days of exposure to an elicitor. For purposes of the present invention the time for maximum yield of metabolites will vary for each cell culture, but can readily be determined by a time course experiment. In soybean cell cultures maximum PAL activity was found 7 to 8 hours after elicitation with a yeast carbohydrate preparation, maximum glyceollin synthesis followed 2 to 3 hours later, i.e. 10 to 12 hours after elicitation. At the same time, for a given cell line, different products may show highest levels of accumulation at different times, i.e., kievitone and phaseollin in Phaseolus vulgarus or phytuberrin and phytuberol in Nicotiana callus cultures.

Elicitors or elicitor preparations include both biotic and abiotic elicitors. Biotic elicitors intends those compounds of biological origin involved in plant-microbe interaction. The biotic elicitors are, in general, low molecular weight components of the microbial cell wall or extra cellular enzymes which induce plants to produce secondary metabolites as a cellular defense mechanism and includes homogenates, filtrates and extracts, including oligosaccharides, polysaccharides, glycoproteins and low molecular weight compounds, of fungal or bacterial origin. Pathogenic fungi, i.e., Phytophthora, Botrytis, Verticillium, etc., and non-pathogenic fungi, i.e. Aspergillus, Micromucor, Rhodotorula, etc. may find use. Bacterial elicitors include Rhizobium, Erwinia, Streptomyces, and the like. Table 1 gives examples of several elicitors as well as their means of preparation, the cell culture upon which they act and the products produced. Cultures show a significant increase in metabolite production after exposure to elicitors (Table 2).

Abiotic elicitors include stress agents such as UV light, alkalinity, osmotic pressure, heavy metal ions, and the like. Thus a wide variety of elicitors are known and can be utilized with the present method. Optimum employment of elicitor preparation for product accumulation requires the consideration of several criteria which may be optimized for each cell culture. These criteria include: elicitor specificity, elicitor concentration, duration of elicitor contact, cell line, time course of elicitation, growth stage of the culture, growth regulation, and nutrient composition.

To determine the most efficient elicitor for any cell culture and particular metabolite, a variety of elicitor preparations may be screened. Certainly, where elicitors are known for a particular culture they may be utilized. The combination of more than one biotic elicitor to the elicitor preparation may find use as well as the combination of biotic and abiotic elicitors.

Protocols for the preparation of elicitors are known, such as is described in Eilert et al, J. Plant Physiol. (1985) 119: 65-76, whose disclosure is herein incorporated by reference. One such process for the preparation of fungal elicitors includes the excision of 1 $cm^2$ mycelium, inoculation of 100 ml of B5-medium without hormones, culture for seven days at room temperature, and homogenization of the entire culture followed with sterilization by autoclaving. Elicitor preparations can be further refined, if desired, by fractionation and purification.

The amount of elicitor preparation which is effective for the enhancement of secondary metabolite production varies widely and can be determined for each cell material. Maxium glyceollin synthesis in soybean cell cultures was obtained with a yeast carbohydrate preparation of 20 mg/g dry weight of cells.

The amount of time after elicitor contact for the accumulation of secondary metabolites will vary depending upon the elicitor preparation, the cell line being cultured, and the product being obtained. For the most part, only a relatively short exposure is necessary when considering products such as phytoalexins and other components of a plant's defense system. After elicitor contact the metabolites can be isolated by triggering the cells to release the stored metabolites into the medium, followed by an exchange of the culture medium, or by cell isolation with subsequent extraction of the metabolites. Thus, in general, elicitor contact will range from about 24 hours to about 60 hours preferably about 48 hours. This relatively shore duration of exposure necessary to induce maximum product accumulation is, indeed, a beneficial trait of elicitation.

Elicitors can be used alone or in combination with other means for increasing secondary metabolite production. These other methods include, but are not limited to, the use of a production medium, the variation of factors having a direct effect on secondary metabolite formation such as hormones or other chemical factors and variation of factors which affect the growth and viability of the cell cultures. It has been demonstrated (see Tables and Experimental Section) that the addition of cell viability stabilizers, and/or nutrients at the time of elicitation are particularly beneficial as they extend cell viability and maintain optimum secondary metabolite production following elicitor stress. Although these components improve the efficiency of metabolite production in general, they are especially important in situations where sudden product formation from elicitation is deleterious to plant cells.

Cell viability stabilizers include polyamines, osmolytes and the like. When polyamines are added along with elicitors, the transient increase in PAL activity following elicitation is stabilized and an increase in the amount of secondary metabolites formed following elicitation is seen (Tables 3 and 4). Polyamines are added for a final concentration of about 2 mM to about 10 mM, preferably about 5 mM. By polyamines is intended cadaverine, putrescine, spermidine, spermine, and the like. The use of tetramines are perhaps preferable as they are more effective than di- or triamines. Polyamines increase in plants following application of plant hormones and perhaps serve as secondary messengers similar to cAMP. Thus, the addition of plant hormones to the culture can have an influence on the growth and product formation of metabolites by increasing polyamines. In general, polyamines regulate physical and chemical properties in membranes and thereby alter permeability.

Organic osmolytes are cytoplasmic components which serve as osmoregulators and perhaps function primarily as protein stabilizers. Organic osmolytes include proline, ergosterol, glycerol, and the like. The addition of organic osmolytes following elicitation results in corresponding increases in secondary metabolite reduction (Tables 5 and 6). Osmolytes are demonstrated to be effective when added for a final concentration of about 0.5% w/v to about 2% w/v, preferably about 1% w/v.

For the purposes of the present invention, nutrients include macronutrients, micronutrients, vitamins, amino acids, hormones, growth regulators, carbohydrate sources, and the like. The variation of or addition of any of these components at the time of elicitation can result in improved yields of secondary metabolites. However, particularly beneficial results have been achieved by the addition of calcium (Table 7) and sucrose and potassium nitrate along with the elicitor. For calcium, a final concentration in the range of 1 mM to about 5 mM, preferably about 2 mM is preferred. For sucrose and potassium nitrate, final concentrations in the range of about 0.5% w/v to about 2% w/v, preferably 1% w/v, and about .05% w/v to about 0.3% w/v, preferably 0.1% w/v respectively, gives optimal results.

The addition of nutrients and/or stabilizers improved metabolite production when utilized independently at the time of elicitation. However, even greater yields may be accumulated by the addition of more than one component at the time of elicitation (see Tables 2 to 8). As discussed, optimum yields were found when cell viability stabilizers, sucrose, potassium nitrate, and the elicitor was added. Therefore, although the addition of elicitors alone at the optimum time in the cell cycle enhances the production of secondary metabolites, significant increases can also be obtained by the utilization of cell viability stabilizers and/or nutrients at the time of elicitation.

The use of a two-stage culturing process using a second state of culturing in a liquid production medium (Zenk et al. In: Plant Tissue Culture and Its Biotechnological Application (1977). Barz et al. (eds.), pp. 27-43 and U.S. Patent No. 4,717,664) has been demonstrated to produce high levels of secondary metabolites in some cell cultures. The instant invention circumvents the need for the second stage of culturing in a production medium. The elicitation process provides for the substantial accumulation of metabolites without the imposition of special culture conditions or the transfer of cells to production media. However, if desired, elicitors may be used in combination with a production media process to shorten the culture period required for maximum product accumulation.

Metabolites, herein, are defined as elicitation products. These include a wide range of ubiquitous plant constituents, such as phenolic compounds, flavonoids, flavones, flavenones, phytoalexins, pterocarpans, alkaloids, and the like. These metabolites find use as proteinase inhibitors, plant virus inhibitors, enzymes, pigments, antitumor substances, plasma inhibitory compounds, antibiotics, spermicides, anti-inflammatory substances, etc. Metabolites or compounds which are attractive from the point of attempting to produce them by cell suspension culture include common and essential drug compounds derived from higher plants such as steroids, codeine, atropine, reserpine, digoxin, morphine, colchicine, etc. Besides these compounds which have been listed, there are of course numerous other compounds of pharmaceutical use which can be produced by the instant method. This includes the development of plant tissue and cell culture fermentation as a source for antineoplastic agents from cells of Maytenus, Brucea, Taxus, Baccharis and the like.

Aside from pharmaceuticals, the method finds use in the production of chemicals used in cosmetics, agrochemicals and natural aroma or flavor components from plant origin. The desired aroma compounds are mostly terpenoids which have one or more chiral centers and possess highly esteemed olfactory and taste properties. Chemical synthesis of these compounds is frequently not possible or at least not economical.

Cell cultures which are suitable for use include any which produce the metabolite of interest and respond to elicitors. Examples of the wide variety of cultures include, but are not limited, to those listed in Table 9.

For the industrialization of plant cell cultures to be realized the yield by which a given product is produced by fermentation per unit time must be economical. Important parameters in this regard are cell yield, product yield and growth. Secondary metabolites of plant cells are generally not excreted into the medium, but are retained and stored in the vacuole. The vacuole poses a natural limit as to the maxiumum yield of a given product to accumulate intracellularly. There are exceptions, however, as the excretion of high quantities of glutathione into the medium has been reported for tobacco cells. Likewise, products were detected in the respective media of periwinkle and poppy cells. While such occurrence could be due to excretion of the products into the media or to leakage due to cell breakdown, analysis of the ultrastructure of the cells following elicitation, revealed the cells to be perfectly intact. This finding points to excretion of the products into the culture medium rather than cell breakdown. Those cell cultures which do not naturally excrete metabolites into the culture meduim can be chemically induced to release their secondary metabolites. Chemical means for triggering the release of metabolites includes sorbitanoleate, taurocholate, dimethylsulfoxide, etc. Where necessary, one or a combination of these compounds can be added to the culture medium to trigger the release of secondary metabolites. Thus, one means for isolation of desired metabolites includes triggering the culture cells after elicitation to release the stored secondary metabolites into the medium. After release into the medium, the metabolites could be recovered without destruction of the culture cells. Thus, a preferred embodiment of the invention involves the cyclic process of cell growth and elicitation. Following elicitation, the cells, if necessary, are chemically triggered to release the elicitation products, the culture medium is collected, the metabolites extracted from the culture medium while permitting the plant cells to recycle followed by re-elicitation and extraction. The cycle is continued until a loss in metabolite formation is realized.

Other means for isolation of secondary metabolites includes isolation of the plant cells from the culture medium by gravity filtration, centrifuation and the like. Lysis of the plant cell membranes yields a concentrated slurry of the products from which the desired compounds can be extracted.

Another isolation embodiment includes the immobilization of cell cultures followed alternatively by treatment with elicitor and removal/exchange of medium for product recovery. As calcium alginate has demonstrated elicitor activity, entrapment of cells in calcium alginate followed by exchange of the medium for product extraction may be employed.

The following examples are offered by way of illustration and not by way of limitation.

## EXPERIMENTAL

## Example 1

### Cell Lines and Culture Conditions

Suspension cultures of soybean cells (Glycine max) were derived from callus generated from soybean hypocotyls. The soybean cells were grown in the dark in Gamborgs B-5 medium with 0.5 ppm 2,4-D and 0.1 ppm of kinetin at 26° C and 125 rpm. Cells were subcultured every 7 days using a 20% (v/v) inoculum. The cells grew in clumps of 15-25 cells and sometimes larger aggregates. All experiments were incubated in 250 mL Erlemeyer flasks containing 100 mL medium. Every sampling was done in triplicate.

## Example 2

### Cell Dry Weight Estimation

Ten ml of actively growing culture was removed every day over the growth period. The sample was filtered under vacuum onto preweighed Whatman 1 filter paper. The paper was then oven dried for 24-36 hours at 70° C before being reweighed to determine the cell dry weight. Cell dry weight values were corrected to g/l of medium. The filtrate was retained and used for determination of peroxidase activity.

## Example 3

### Cell Dry Weight to Fresh Weight Correlation

About 70 samples from various stages of the growth cycle were measured for both their dry weight, which was determined gravimetrically prior to transferring samples to the oven. The correlation of dry weight to wet weight showed an average of 4.8% of fresh weight is made up of cell dry weight (60% of the estimations fell in the range of 4.6% - 4.9%).

## Example 4

### Peroxidase activity

Peroxidase activity was determined and correlated to cell dry weight. 4-Aminoantipyrine was used as the hydrogen donor in the peroxidase assay. The reaction rate was determined by measuring an increase in absorbance at 510 nm resulting from the decomposition of hydrogen peroxide and oxidation of the pyrine.

Procedure: 1.4 mL of phenol/antipyrine solution and 1.5 ml of 0.0017M hydrogen peroxide were mixed. The solution was then incubated for 3-4 minutes to achieve temperature equilibrium and establish the background reaction rate. 0.1 ml of extracellular filtrate (enzyme) was then added and the increase in absorbance at 510 nm was followed for 4-5 minutes. Activity was expressed as $A_{510}$/minute.

Example 5

Phenolic determination

0.5g (fresh wt %) cells (equivalent to 24 mg dry weight as determined by the correlation above) in 5 ml of 95% ethanol was sonically disrupted for 3-5 minutes. Afterwards the contents were centrifuged for 30 minutes at 12,000 rpm in a Beckman Model J21C centrifuge. The ethanol fraction (supernatant) was retained for the phenolic assay.

Assay: 1 ml of ethanol extract and 1 ml of 75% ethanol were mixed in 5 ml distilled water. then, 0.5 ml of 50% Folin-Ciocalteau reagent was added. After 5 min, 1 ml of 5% $Na_2CO_3$ was added, and the reaction mixture was allowed to stand for 60 min. Its absorbance was then measured at 725 nm. Controls contained only 95% ethanol. A standard curve was developed using various concentrations of gallic acid in 95% ethanol.

Example 6

Carbohydrate Estimation

One ml of cell free medium was assayed for carbohydrates by the anthrone method, Morris (1948) Science (1948) 107: 254. Carbohydrate depletion was estimated by measuring changes in absorbance of the anthrone color at 540 nm, from a standard curve using sucrose as the standard carbohydrate. Total carbohydrate in culture supernatants was expressed as grams of carbohydrate per liter of medium.

Example 7

Phenylalanine Ammonia Lyase Activity (PAL)

One gram of cells was suspended in 5 ml of pH 8.8 borate-HCl extraction buffer (25 mM borate-HCl + 2 mM sodium bisulfate). The suspension was gently ground with a mortar and pestle, the resulting mixture was sonically disrupted (1 minute; repeated 4-5 times at approximately 0°C). Between sonic disruptions the probe was cooled with ice for 30 seconds. After sonication, the homogenates were centrifuged at 10,000 rpm for 20 minutes, and the supernatant was then used as the enzyme extract.

Assay: 15 $\mu$M L-phenylalanine in 100 $\mu$M borate buffer (pH8.8) in a total volume of 2.8 ml was mixed with 0.2 ml of enzyme extract and incubated at 30°C. Controls contained 2.8 ml of 100 $\mu$M Borate HCl (pH 8.8) and 0.2 ml enzyme extract with no L-phenylalanine. During the first 30 minutes of incubation, there was a slow decrease in absorbance (290 nm) during stabilization of the reaction mixture. After stabilization, absorbance increase was followed at 290 nm for 60 minutes. Every assay was done in triplicate and corrected by subtraction of the corresponding control value. PAL activity was reported as milliunits (mu), where 1 mu equalled an absorbance change of 0.001 per minute per gram fresh weight of cells.

## Example 8

### Elicitors

Fusarim solani var. pisi.: One liter of late logarithmic phase cells grown in potato dextrose medium were centrifuged and, after determining their fresh weight, the cells were disrupted by blending and resuspended in 25 ml of 100mM potassium acetate (pH 5.5) in a 50 ml flask. This suspension was autoclaved (121 °C; 15 min.). A 0.05% w/v equivalent of sterile elicitor was added to cell suspension cultures.

### Microbial Cell Extracts as Elicitors in Phenolics Production

To obtain higher product concentration in plant cell cultures, separate growth media and product formation media can be used. We have circumvented this need and also reduced the time during which the optimum quantity of phenolics are produced by using microbial elicitors (Table 2). In this study cell extracts of fungus Fusarium solani var pisi, when added at the end of exponential growth (7 days) produced optimum phenolics in 8 days. L-Phenylalanine ammonia lyase activity transiently increased at 6 hours following elicitor addition.

## Example 9

### Growth and Phenolic Production

Growth parameters during exponential growth were determined using the equation:
$X = X_o e^{\mu max(t-to)}$ where X is dry weight. Growth in batch culture was exponential from days 1 to 6 after one day lag phase. The lag phase was short due to the high inoculum level (20% v/v). The specific growth rate ($\mu_{max}$) in the exponential phase was 0.21 day$^{-1}$ corresponding to a doubling time of 3.3 days. Before the onset of stationary phase, the culture entered a declining growth rate period between 6-9 days. This declining growth rate was also reflected in a declining rate of carbohydrate utilization. The rate of carbohydrate utilization was rapid and approximately linear from the time of inoculation to day 7. After day 7, the rate of carbohydrate utilization shifted from 2.3 g/l-day to 1.0 g/l-day.

Ethanol soluble phenolics were produced in approximately equal amounts during the growth and the stationary phases. There was little phenolics production in the declining growth phase and after day 12. After an additional 3 days, the phenolics concentration had increased only 15% from the level observed at day 12.

## Example 10

### Extracellular Peroxidase and L-Phenylalanine Ammonia Lyase (PAL Activity during Growth)

The rate of extracellular peroxidase activity as an indicator of cell growth was also examined. It was found that peroxidase activity was correlated with growth. The leveling off of peroxidase activity coincided with the changes in growth kinetics, rate of carbohydrate utilization, and phenolic accumulation. A most interesting observation was that optimum phenolic formation as a result of elicitation was triggered when elicitors were added at the end of 7 days of cell growth. Adding elicitor earlier than 7 days was not feasible because of low cell mass, or beyond 7 days because the enzymes needed to respond to elicitors and turn on phenolic production had reduced activities, as exemplified by the peroxidase activity. Also, with the onset of senescence, there was reduced cell-division as cells entered stationary phase.

L-Phenylalanine ammonia-lyase (PAL) activity was followed over the growth period. PAL activity increased at the onset of stationary phase coinciding with the onset of soluble phenolics production. PAL is a key enzyme of the phenylpropanoid pathway and many of these compounds are formed when cell division ceases.

Example 11

Role of Calcium, Polyamines and Osmolytes in Cellular Production of Phenolics

Calcium: Calcium increased activity of PAL and concomitantly increased total phenolics when added along with the elicitor (Table 3-7). The transient increase in PAL was similar to what was observed by elicitor alone though overall activity was higher. Changes in calcium concentration affected the elicitor mediated phytoalexin accumulation, and by removal of all calcium with chelating agents, elicitor mediated enzyme induction and phytoalexin production were eliminated.

Polyamines The polyamines spermidine and spermine when added along with calcium and elicitors stabilized the transient increase in PAL activity following elicitation. They also increased the amount of phenolics formed compared to using only calcium and elicitors (Tables 3 and 4). It appears that the quality of phenolics formed may be affected by polyamines. It has been observed that spermidine not only extended the life span of mature non-dividing cells but also increased yields of phenolics and gave a wider assortment of phenolics, Muhitch et al (1985) Plant Physiol (1985) 78: 25.

Organic Osmolytes: Organic osmolytes are cytoplasmic components serving as osmoregulators and perhaps working primarily as protein stabilizers. The organic osmolyte proline used in this study did not stabilize the transient increase in PAL activity following elicitor and calcium addition. However, proline gave an increase in phenolics produced comparable to adding elicitor and calcium alone. (Tables 5 and 6). The organic osmolyte glycerol stabilized the transient increase in PAL activity following elicitor addition. The addition of glycerol also resulted in a corresponding increase in phenolics (Table 6).

Addition of Sucrose and a Nitrogen Source During Elicitation for Phenolics Production: Phenolics production in Glycine max cell suspension was highest when elicitor was added with a cell viability stabilizer, sucrose and potassium nitrate (Table 7).

The instant method offers several advantages for the production of secondary metabolites through cell culture. Current methods for the production of secondary metabolites requires culturing the cells first in a growth media followed by culturing in a product formation media. The present invention circumvents this need for a growth media and production formation media. The use of only one culture media reduces labor and cost of the production of metabolites, as well as reduces the time during which the optimum quantity of metabolites are produced.

The use of elicitors to enhance secondary metabolite formation offers a powerful tool for the production of specific compounds through tissue culture. Improved yields of metabolites are obtained by elicitation alone. Further maximization can be realized by the addition of cell viability stabilizers and/or nutrients at the time of elicitation.

All publications and patent applications mentioned in this specification are indicative of the level of skill of those skilled in the art to which this invention pertains. All publications and patent applications are herein incorporated by reference to the same extent as if each individual publication or patent appliction was specifically and individually indicated to be incorporated by reference.

Although the foregoing invention has been described in some detail by way of illustration and example for purposes of clarity of understanding, it will be obvious that certain changes and modifications may be practiced within the scope of the appended claim.

Table 1

| ELICITOR-INDUCED PRODUCT ACCUMULATION | | | |
|---|---|---|---|
| Elicitor | Preparation | Cell Culture | Products |
| Pythium aphanidermatum<br>Eurotium rubrum<br>Micromucor isabellina<br>Chrysosporium palmorum | Filtrates and extracts | Catharanthus roseus | Tryptamine<br>Ajmalicine<br>Catharanthine |
| Pythium aphanidermatum | Homogenate | Catharanthus roseus | Strictosidine<br>Ajmalicine<br>Tabersonine<br>Lochmericine<br>Catharanthine |
| Botrytis spec. | Homogenate | Papaver somniferum | Sanguinarine |
| Dendryphion spec. | Extract | Papaver somniferum | Sanguinarine |
|  |  | Papaver bacteatum | Sanguinarine |
| Yeast | Carbohydrate preparation | Glycine max | Glyceollin |
|  |  | Thalictrum rugosum | Berberine |
| Aspergillus niger | Homogenate | Cinchona ledgeriana<br>Rubia tinctoria<br>Morinda citrofolia | Anthraquinones |
| Nigeran |  | Solanum melongena<br>Vigna angularis | Polyacetylenes<br>Isoflavones |

Table 2

| Elicitor Responses as Measured by Soluble Phenolics (mg/l) after Addition of Microbial Elicitors ( Fusarim solani var. pisi) | | | |
|---|---|---|---|
|  | Time (h) | | |
|  | 0 | 24 | 48 |
| Elicitor | 61.7 | 123.1 | 137.3[a] |
| Control (no addition) | 63.4 | 62.1 | 67.4 |

[a]Significant at 95% confidence level from all other treatments using Fisher's L.S.D. All values are average of three readings.

Table 3

| Elicitor Responses as Measured by Soluble Phenolics (mg/l) after Addition of Microbial Elicitors: ( Fusarium solani var. pisi) | | | |
|---|---|---|---|
| Effect of Calcium (2 mM) and Spermidine (5 mM) | | | |
| | Time (h) | | |
| | 0 | 24 | 48 |
| Elicitor + $Ca^{++}$ + Spermidine | 63.7 | 127.8 | 158.6[a] |
| Elicitor + $Ca^{++}$ | 61.8 | 118.7 | 143.6 |
| Elicitor + Spermidine | 63.9 | 116.3 | 141.3 |
| Elicitor | 64.6 | 123.6 | 133.7 |
| Control + $Ca^{++}$ + Spermidine | 59.2 | 63.4 | 62.7 |
| Control + $Ca^{++}$ | 67.3 | 64.6 | 65.3 |
| Control + Spermidine | 58.6 | 61.4 | 63.6 |
| Control | 63.7 | 61.6 | 62.3 |

[a]Significant at 95% confidence level from all other treatments using Fisher's L.S.D. All values are average of three readings.

Table 4

| Elicitor Responses as Measured by Soluble Phenolics (mg/l) after Addition of Microbial Elicitors: ( Fusarium solani var. pisi) | | | |
|---|---|---|---|
| Effect of Calcium (2 mM) and Spermine (5 mM) | | | |
| | Time (h) | | |
| | 0 | 24 | 48 |
| Elicitor + $Ca^{++}$ + Spermine | 72.3 | 123.7 | 158.6[a] |
| Elicitor + $Ca^{++}$ | 69.6 | 124.2 | 143.3 |
| Elicitor + Spermine | 63.7 | 118.7 | 140.8 |
| Elicitor | 68.7 | 120.3 | 131.4 |
| Control + $Ca^{++}$ + Spermine | 63.6 | 70.4 | 69.8 |
| Control + $Ca^{++}$ | 64.8 | 67.3 | 68.1 |
| Control + Spermine | 61.2 | 62.8 | 70.3 |
| Control | 69.1 | 67.4 | 62.8 |

[a]Significant at 95% confidence level from all other treatments using Fisher's L.S.D. All values are average of three readings.

Table 5

| Elicitor Responses as Measured by Soluble Phenolics (mg/l) after Addition of Microbial Elicitors: ( Fusarium solani var. pisi) | | | |
|---|---|---|---|
| Effect of Calcium (2 mM) and Proline (5 mM) | | | |
| | Time (h) | | |
| | 0 | 24 | 48 |
| Elicitor + $Ca^{++}$ + Proline | 71.4 | 117.3 | 158.1[a] |
| Elicitor + $Ca^{++}$ | 63.8 | 119.7 | 141.6 |
| Elicitor + Proline | 61.3 | 118.8 | 147.3 |
| Elicitor | 63.7 | 121.7 | 138.8 |
| Control + $Ca^{++}$ + Proline | 69.8 | 63.8 | 64.3 |
| Control + $Ca^{++}$ | 63.9 | 65.6 | 67.8 |
| Control + Proline | 69.8 | 69.3 | 68.4 |
| Control | 70.6 | 71.4 | 72.3 |

[a]Significant at 95% confidence level from all other treatments using Fisher's L.S.D. All values are average of three readings.

Table 6

| Elicitor Responses as Measured by Soluble Phenolics (mg/l) after Addition of Microbial Elicitors: ( Fusarium solani var. pisi) | | | |
|---|---|---|---|
| Effect of Calcium (2 mM) and Glycerol (1% w/v) | | | |
| | Time (h) | | |
| | 0 | 24 | 48 |
| Elicitor + $Ca^{++}$ + Glycerol | 53.4 | 123.7 | 160.4[a] |
| Elicitor + $Ca^{++}$ | 54.3 | 119.3 | 143.3 |
| Elicitor + Glycerol | 59.8 | 129.6 | 153.3 |
| Elicitor | 57.4 | 123.8 | 141.7 |
| Control + $Ca^{++}$ + Glycerol | 61.8 | 63.8 | 67.4 |
| Control + $Ca^{++}$ | 59.6 | 61.7 | 62.2 |
| Control + Glycerol | 57.6 | 59.3 | 58,4 |
| Control | 63.4 | 61.8 | 67,6 |

[a]Significant at 95% confidence level from all other treatments using Fisher's L.S.D. All values are average of three readings.

Table 7

| Elicitor Responses as Measured by Soluble Phenolics (mg/l) after Addition of Microbial Elicitors: ( Fusarium solani var. pisi) | | | |
|---|---|---|---|
| Effect of Calcium ($Ca^{++}$) | | | |
| | Time (h) | | |
| | 0 | 24 | 48 |
| Elicitor | 63.4 | 117.4 | 139.8 |
| $Ca^{++}$ | 61.3 | 63.4 | 64.6 |
| Elicitor + $Ca^{++}$ | 59.7 | 123.7 | 149.8[a] |
| Control (no addition) | 58.7 | 61.3 | 62.7 |

[a]Significant at 95% confidence level from all other treatments using Fisher's L.S.D. All values are average of three readings.

Table 8

| Elicitor Responses as Measured by Soluble Phenolics (mg/l) after Addition of Microbial Elicitors: ( Fusarium solani var. pisi) | | |
|---|---|---|
| Effect of Calcium, Polyamines/Osmolytes and Sucrose + $KNO_3$ | | |
| | Time (h) | |
| | 0 | 24 |
| Control | 57.8 | 64.7 |
| Sucrose + $KNO_3$ | 68.4 | 63.4 |
| Elicitor + (Sucrose + $KNO_3$) | 63.4 | 154.7 |
| Elicitor + $Ca^{++}$ + (Sucrose + $KNO_3$) | 59.3 | 159.8 |
| Elicitor + $Ca^{++}$ + (Sucrose + $KNO_3$) + Spermine | 63.3 | 160.4 |
| Elicitor + $Ca^{++}$ + (Sucrose + $KNO_3$) + Spermidine | 59.5 | 171.3[a] |
| Elicitor + $Ca^{++}$ + (Sucrose + $KNO_3$) + Proline | 65.4 | 159.7 |
| Elicitor + $Ca^{++}$ + (Sucrose + $KNO_3$) + Glycerol | 59.6 | 174.6[a] |

[a]Significant at 95% confidence level from all other treatments using Fisher's L.S.D. All values are average of three readings.

Table 9

| Metabolites and the Cell Cultures From Which They Are Produced | |
| --- | --- |
| Product | Plant Species |
| Ginsengoside | Panax ginseng |
| Anthraquinones | Morinda citrifolia |
| Rosmarinic acid | Coleus blumei |
| Shikonin | Lithospermum erythrorhizon |
| Anthraquinones | Cassia tora |
| Diosgenin | Dioscorea deltoides |
| Biscoclaurine | Stephania cepharantha |
| Caffein | Coffea arabica |
| Ajmalicine | Catharanthus roseus |
| Paniculide B | Andrographis paniculate |
| Serpentine | Catharanthus roseus |
| Protopine | Macleaya microcarpa |
| Visnagin | Ammi visnaga |
| Glutathione | Nicotiana tabacum |
| Ubiquinone-10 | Nicotiana tabacum |
| Alkaloids | Catharanthus roseus |
| Nicotine | Nicotiana rustica |
| Alkaloids | Solanum laciniatum |
| Catechols | Ilex pupescens |
| Catechols | Artemisia capillaris |
| Catechols | Salvia miltiorrhiza |
| Artemisian | Artemisia annua |
| Betacyanin | Beta vulgaris |
| Tannin | Krameria triandra |
| Tannin | Archtostaphylos upa-uris |
| Cinnamic acid derivatives | Artemesia tridentata |
| Berberine | Coptis japonica |
| Vanillin | Vanilla planifolia |

## Claims

1. A method for enhancing secondary metabolite production from a plant cell culture, said method comprising:
growing a plant cell culture in a production medium to about the end of exponential growth phase;
adding to said medium an elicitor preparation in an amount to enhance secondary metabolite production; and
isolating at least one secondary metabolite.

2. A method, according to Claim 1, wherein said end of exponential growth phase is determined by monitoring extracellular products.

3. A method, according to Claim 2, wherein said monitoring comprises assaying for extracellular peroxidase activity.

4. A method for enhancing secondary metabolite production from a plant cell culture, said method comprising:
growing a plant cell culture in a production medium to at least about the end of exponential growth phase;
adding to said medium an elicitor preparation and at least one of cell viability stabilizers and nutrients in amounts to enhance and stabilize secondary metabolite production; and,
isolating at least one secondary metabolite.

5. A method, according to Claim 4, wherein said end of exponential growth phase is determined by

15

assaying for extracellular peroxidase activity.

6. A method, according to claim 4 or claim 5, wherein said viability stabilizers comprise polyamines and osmolytes.

7. A method, according to Claim 6, wherein said polyamines are selected from spermidine, spermine, cadaverine, and putrescine.

8. A method, according to Claim 7, wherein said polyamine is spermidine.

9. A method, according to Claim 7, wherein said polyamine is spermine.

10. A method, according to Claim 6, wherein said osmolytes are selected from proline, ergosterol and glycerol.

11. A method, according to Claim 10, wherein said osmolyte is proline.

12. A method, according to Claim 10, wherein said osmolyte is glycerol.

13. A method, according to claim 4 or claim 5, wherein said nutrients are selected from sucrose, calcium, and potassium nitrate.

14. A method, according to claim 4 or claim 5, wherein said adding step comprises the addition of an elicitor preparation, calcium, sucrose, postassium nitrate and at least one of spermine, spermidine, proline and glycerol.

15. A method, according to any one of the preceding claims wherein said secondary metabolite is flavonoids, flavones, flavonones, phytoalexins, pterocarpins, and alkaloids.

16. A method, according to any one of the preceding claims, wherein said plant cell cultures are produced from plants of Boraginaceae, Pyrolaceae, Juglandaceae, Plumbaginaceae, Lythraceae, Ranunculaceae and Solanaceae.

17. A method, according to Claim 16, wherein said secondary metabolite is artemesian and said plant cell culture is Artemesia annua.

18. A method, according to Claim 16, wherein said secondary metabolite is betacyanin and said plant cell culture is Beta vulgaris.

19. A method, according to Claim 16, wherein said secondary metabolite is vanillin and said plant cell culture is Vanilla planifolia.

20. A method, according to Claim 16, wherein said secondary metabolite is rosmarinic acid and said plant cell culture is Coleus blumei.